# EUROPEAN PATENT SPECIFICATION

(11) **EP 1 092 017 B1**
(45) Date of publication and mention of the grant of the patent: **14.11.2007**
(21) Application number: 99928897.0
(22) Date of filing: 29.06.1999
(51) Int. Cl.: C12N 15/11, C07K 14/705, C07K 16/28

(54) **NOVEL GABA-B RECEPTOR**
NEUER GABA-B REZEPTOR
RECEPTEUR GABA-B

(30) Priority: 29.06.1998 AU PP438498
(43) Date of publication of application: 18.04.2001
(73) Proprietor: GARVAN INSTITUTE OF MEDICAL RESEARCH, Darlinghurst, NSW 2010 (AU)
(72) Inventor: HERZOG, Herbert, Bondi, NSW 2026 (AU)
(74) Representative: Maschio, Antonio
(86) International application number: PCT/AU1999/000524
(87) International publication number: WO 2000/000602

(56) References cited:
- WO-A-97/46675
- WO-A-98/45437
- WO-A-99/40114
- WO-A-99/42603
- WO-A-99/51636
- WO-A-99/61606
- GENPEPT, Accession Number AAD30389 Submitted 15 November 1998, by LUI M. et al.
- NATURE, 396, (1998), by WHITE J.H. et al., "Heterodimerization is Required for the Formation of a Functional GABA(B) Receptor", pages 679-682.
- EMBL, Accession Number AF095784, Submitted 17 May 1999, by LUI M. et al.
- EMBL, Accession Number AF069755, Submitted 4 January 1999, by N.G. GYK et al.
- EMBL, Accession Number AJ012188, Submitted 16 October 1998, by FRASER N.J.
- GENPEPT, Accession Number CAA09942, Submitted 16 October 1998, by WHITE J.H. et al.
- EMBL, Accession Number AF074483, Submitted 25 June 1998, by BOROWSKY B. et al.
- EMBL, Accession Number AF056085, Submitted 27 March 1998, by CLARK J.C. et al.
- CURRENT OPINION IN NEUROBIOLOGY, 8(3), (June 1998), by BETTLER B. et al., "GABAB Receptors Drugs Meet Clones", pages 345-350.

### SEQUENCE LISTING

<110> Garvan Institute of Medical Research
<120> Novel GABA-B Receptor
<130> P010281EP CYK
<140> 99928897.0
   <141> 1999-06-29
<150> PP4384
   <151> 1998-06-29
<160> 4
<170> Patent In version 3.0
<210> 1
   <211> 10
   <212> PRT
   <213> Homo sapiens
<400> 1
   Met Ala Ser Pro Arg Ser Ser Gly Gln Pro
   1               5                   10
<210> 2
   <211> 941
   <212> PRT
   <213> Homo sapiens
<400> 2
   Met Ala Ser Pro Arg Ser Ser Gly Gln Pro Gly Pro Pro Pro Pro Pro
   1               5                   10                  15
   Pro Pro Pro Pro Ala Arg Leu Leu Leu Leu Leu Leu Leu Pro Leu Leu
               20                  25                  30
   Leu Pro Leu Ala Pro Gly Ala Trp Gly Trp Ala Arg Gly Ala Pro Arg
   35 40 45
   Pro Pro Pro Ser Ser Pro Pro Leu Ser Ile Met Gly Leu Met Pro Leu
   50 55 60
   Thr Lys Glu Val Ala Lys Gly Ser Ile Gly Arg Gly Val Leu Pro Ala
   65 70 75 80
   Val Glu Leu Ala Ile Glu Gln Ile Arg Asn Glu Ser Leu Leu Arg Pro
   85 90 95
   Tyr Phe Leu Asp Leu Arg Leu Tyr Asp Thr Glu Cys Asp Asn Ala Lys
   100 105 110
   Gly Leu Lys Ala Phe Tyr Asp Ala Ile Lys Tyr Gly Pro Asn His Leu
   115 120 125
   Met Val Phe Gly Gly Val Cys Pro Ser Val Thr Ser Ile Ile Ala Glu
   130 135 140
   Ser Leu Gln Gly Trp Asn Leu Val Gln Leu Ser Phe Ala Ala Thr Thr
   145 150 155 160
   Pro Val Leu Ala Asp Lys Lys Lys Tyr Pro Tyr Phe Phe Arg Thr Val
   165 170 175
   Pro Ser Asp Asn Ala Val Asn Pro Ala Ile Leu Lys Leu Leu Lys His
   180 185 190
   Tyr Gln Trp Lys Arg Val Gly Thr Leu Thr Gln Asp Val Gln Arg Phe
   195 200 205
   Ser Glu Val Arg Asn Asp Leu Thr Gly Val Leu Tyr Gly Glu Asp Ile
   210 215 220
   Glu Ile Ser Asp Thr Glu Ser Phe Ser Asn Asp Pro Cys Thr Ser Val
   225 230 235 240
   Lys Lys Leu Lys Gly Asn Asp Val Arg Ile Ile Leu Gly Gln Phe Asp
   245 250 255
   Gln Asn Met Ala Ala Lys Val Phe Cys Cys Ala Tyr Glu Glu Asn Met
   260 265 270
   Tyr Gly Ser Lys Tyr Gln Trp Ile Ile Pro Gly Trp Tyr Glu Pro Ser
   275 280 285
   Trp Trp Glu Gln Val His Thr Glu Ala Asn Ser Ser Arg Cys Leu Arg
   290 295 300
   Lys Asn Leu Leu Ala Ala Met Glu Gly Tyr Ile Gly Val Asp Phe Glu
   305 310 315 320
   Pro Leu Ser Ser Lys Gln Ile Lys Thr Ile Ser Gly Lys Thr Pro Gln
   325 330 335
   Gln Tyr Glu Arg Glu Tyr Asn Asn Lys Arg Ser Gly Val Gly Pro Ser
   340 345 350
   Lys Phe His Gly Tyr Ala Tyr Asp Gly Ile Trp Val Ile Ala Lys Thr
   355 360 365
   Leu Gln Arg Ala Met Glu Thr Leu His Ala Ser Ser Arg His Gln Arg
   370 375 380
   Ile Gln Asp Phe Asn Tyr Thr Asp His Thr Leu Gly Arg Ile Ile Leu
   385 390 395 400
   Asn Ala Met Asn Glu Thr Asn Phe Phe Gly Val Thr Gly Gln Val Val
   405 410 415
   Phe Arg Asn Gly Glu Arg Met Glu Thr Ile Lys Phe Thr Gln Phe Gln
               420                 425                 430
   Asp Ser Arg Glu Val Lys Val Gly Glu Tyr Asn Ala Val Ala Asp Thr
           435                 440                 445
   Leu Glu Ile Ile Asn Asp Thr Ile Arg Phe Gln Gly Ser Glu Pro Pro
       450                 455                 460
   Lys Asp Lys Thr Ile Ile Leu Glu Gln Leu Arg Lys Ile Ser Leu Pro
   465                 470                 475                 480
   Leu Tyr Ser Ile Leu Ser Ala Leu Thr Ile Leu Gly Met Ile Met Ala
                   485                 490                 495
   Ser Ala Phe Leu Phe Phe Asn Ile Lys Asn Arg Asn Gln Lys Leu Ile
               500                 505                 510
   Lys Met Ser Ser Pro Tyr Met Asn Asn Leu Ile Ile Leu Gly Gly Met
           515                 520                 525
   Leu Ser Tyr Ala Ser Ile Phe Leu Phe Gly Leu Asp Gly Ser Phe Val
       530                 535                 540
   Ser Glu Lys Thr Phe Glu Thr Leu Cys Thr Val Arg Thr Trp Ile Leu
   545                 550                 555                 560
   Thr Val Gly Tyr Thr Thr Ala Phe Gly Ala Met Phe Ala Lys Thr Trp
                   565                 570                 575
   Arg Val His Ala Ile Phe Lys Asn Val Lys Met Lys Lys Lys Ile Ile
               580                 585                 590
   Lys Asp Gln Lys Leu Leu Val Ile Val Gly Gly Met Leu Leu Ile Asp
           595                 600                 605
   Leu Cys Ile Leu Ile Cys Trp Gln Ala Val Asp Pro Leu Arg Arg Thr
       610                 615                 620
   Val Glu Lys Tyr Ser Met Glu Pro Asp Pro Ala Gly Arg Asp Ile Ser
   625                 630                 635                 640
   Ile Arg Pro Leu Leu Glu His Cys Glu Asn Thr His Met Thr Ile Trp
                   645                 650                 655
   Leu Gly Ile Val Tyr Ala Tyr Lys Gly Leu Leu Met Leu Phe Gly Cys
               660                 665                 670
   Phe Leu Ala Trp Glu Thr Arg Asn Val Ser Ile Pro Ala Leu Asn Asp
           675                 680                 685
   Ser Lys Tyr Ile Gly Met Ser Val Tyr Asn Val Gly Ile Met Cys Ile
       690                 695                 700
   Ile Gly Ala Ala Val Ser Phe Leu Thr Arg Asp Gln Pro Asn Val Gln
   705                 710                 715                 720
   Phe Cys Ile Val Ala Leu Val Ile Ile Phe Cys Ser Thr Ile Thr Leu
                   725                 730                 735
   Cys Leu Val Phe Val Pro Lys Leu Ile Thr Leu Arg Thr Asn Pro Asp
               740                 745                 750
   Ala Ala Thr Gln Asn Arg Arg Phe Gln Phe Thr Gln Asn Gln Lys Lys
           755                 760                 765
   Glu Asp Ser Lys Thr Ser Thr Ser Val Thr Ser Val Asn Gln Ala Ser
       770                 775                 780
   Thr Ser Arg Leu Glu Gly Leu Gln Ser Glu Asn His Arg Leu Arg Met
   785                 790                 795                 800
   Lys Ile Thr Glu Leu Asp Lys Asp Leu Glu Glu Val Thr Met Gln Leu
                   805                 810                 815
   Gln Asp Thr Pro Glu Lys Thr Thr Tyr Ile Lys Gln Asn His Tyr Gln
               820                 825                 830
   Glu Leu Asn Asp Ile Leu Asn Leu Gly Asn Phe Thr Glu Ser Thr Asp
           835                 840                 845
   Gly Gly Lys Ala Ile Leu Lys Asn His Leu Asp Gln Asn Pro Gln Leu
       850                 855                 860
   Gln Trp Asn Thr Thr Glu Pro Ser Arg Thr Cys Lys Asp Pro Ile Glu
   865                 870                 875                 880
   Asp Ile Asn Ser Pro Glu His Ile Gln Arg Arg Leu Ser Leu Gln Leu
                   885                 890                 895
   Pro Ile Leu His His Ala Tyr Leu Pro Ser Ile Gly Gly Val Asp Ala
               900                 905                 910
   Ser Cys Val Ser Pro Cys Val Ser Pro Thr Ala Ser Pro Arg His Arg
           915                 920                 925
   His Val Pro Pro Ser Phe Arg Val Met Val Ser Gly Leu
       930                 935                 940
<210> 3
   <211> 3256
   <212> DNA
   <213> Homo sapiens
<400> 3
   gaattccgac gggcggtgtg tacaaagggc agggacttaa tcaacgcaag cttatgaccc     60
   gcactccttg gcgcggggcg gcgggccggg ccaggccatg cgggccgagt gagccggcgc    120
   ccgcagcccg cggcgcggca tggcttcccc gcggagctcc gggcagcccg ggccgccgcc    180
   gccgccgcca ccgccgcccg cgcgcctgct actgctactg ctgctgccgc tgctgctgcc    240
   tctggcgccc ggggcctggg gctgggcgcg gggcgccccc cggccgccgc ccagcagccc    300
   gccgctctcc atcatgggcc tcatgccgct caccaaggag gtggccaagg gcagcatcgg    360
   gcgcggtgtg ctccccgccg tggaactggc catcgagcag atccgcaacg agtcactcct    420
   gcgcccctac ttcctcgacc tgcggctcta tgacacggag tgcgacaacg caaaagggtt    480
   gaaagccttc tacgatgcga taaaatacgg gccgaaccac ttgatggtgt ttggaggcgt    540
   ctgtccatcc gtcacatcca tcattgcaga gtccctccaa ggctggaatc tggtgcagct    600
   ttcttttgct gcaaccacgc ctgttctagc cgataagaaa aaataccctt atttctttcg    660
   gaccgtccca tcagacaatg cggtgaatcc agccattctg aagttgctca agcactacca    720
   gtggaagcgc gtgggcacgc tgacgcaaga cgttcagagg ttctctgagg tgcggaatga    780
   cctgactgga gttctgtatg gcgaggacat tgagatttca gacaccgaga gcttctccaa    840
   cgatccctgt accagtgtca aaaagctgaa ggggaatgat gtgcggatca tccttggcca    900
   gtttgaccag aatatggcag caaaagtgtt ctgttgtgca tacgaggaga acatgtatgg 960
   tagtaaatat cagtggatca ttccgggctg gtacgagcct tcttggtggg agcaggtgca 1020
   cacggaagcc aactcatccc gctgcctccg gaagaatctg cttgctgcca tggagggcta 1080
   cattggcgtg gatttcgagc ccctgagctc caagcagatc aagaccatct caggaaagac 1140
   tccacagcag tatgagagag agtacaacaa caagcggtca ggcgtggggc ccagcaagtt 1200
   ccacgggtac gcctacgatg gcatctgggt catcgccaag acactgcaga gggccatgga 1260
   gacactgcat gccagcagcc ggcaccagcg gatccaggac ttcaactaca cggaccacac 1320
   gctgggcagg atcatcctca atgccatgaa cgagaccaac ttcttcgggg tcacgggtca 1380
   agttgtattc cggaatgggg agagaatgga gaccattaaa tttactcaat ttcaagacag 1440
   cagggaggtg aaggtgggag agtacaacgc tgtggccgac acactggaga tcatcaatga 1500
   caccatcagg ttccaagggt ccgaaccacc aaaagacaag accatcatcc tggagcagct 1560
   gcggaagatc tccctacctc tctacagcat cctctctgcc ctcaccatcc tcgggatgat 1620
   catggccagt gcttttctct tcttcaacat caagaaccgg aatcagaagc tcataaagat 1680
   gtcgagtcca tacatgaaca accttatcat ccttggaggg atgctctcct atgcttccat 1740
   atttctcttt ggccttgatg gatcctttgt ctctgaaaag acctttgaaa cactttgcac 1800
   cgtcaggacc tggattctca ccgtgggcta cacgaccgct tttggggcca tgtttgcaaa 1860
   gacctggaga gtccacgcca tcttcaaaaa tgtgaaaatg aagaagaaga tcatcaagga 1920
   ccagaaactg cttgtgatcg tggggggcat gctgctgatc gacctgtgta tcctgatctg 1980
   ctggcaggct gtggaccccc tgcgaaggac agtggagaag tacagcatgg agccggaccc 2040
   agcaggacgg gatatctcca tccgccctct cctggagcac tgtgagaaca cccatatgac 2100
   catctggctt ggcatcgtct atgcctacaa gggacttctc atgttgttcg gttgtttctt 2160
   agcttgggag acccgcaacg tcagcatccc cgcactcaac gacagcaagt acatcgggat 2220
   gagtgtctac aacgtgggga tcatgtgcat catcggggcc gctgtctcct tcctgacccg 2280
   ggaccagccc aatgtgcagt tctgcatcgt ggctctggtc atcatcttct gcagcaccat 2340
   caccctctgc ctggtattcg tgccgaagct catcaccctg agaacaaacc cagatgcagc 2400
   aacgcagaac aggcgattcc agttcactca gaatcagaag aaagaagatt ctaaaacgtc 2460
   cacctcggtc accagtgtga accaagccag cacatcccgc ctggagggcc tacagtcaga 2520
   aaaccatcgc ctgcgaatga agatcacaga gctggataaa gacttggaag aggtcaccat 2580
   gcagctgcag gacacaccag aaaagaccac ctacattaaa cagaaccact accaagagct 2640
   caatgacatc ctcaacctgg gaaacttcac tgagagcaca gatggaggaa aggccatttt 2700
   aaaaaatcac ctcgatcaaa atccccagct acagtggaac acaacagagc cctctcgaac 2760
   atgcaaagat cctatagaag atataaactc tccagaacac atccagcgtc ggctgtccct 2820
   ccagctcccc atcctccacc acgcctacct cccatccatc ggaggcgtgg acgccagctg 2880
   tgtcagcccc tgcgtcagcc ccaccgccag cccccgccac agacatgtgc caccctcctt 2940
   ccgagtcatg gtctcgggcc tgtaagggtg ggaggcctgg cccgggcctc ccccgtgaca 3000
   gaaccacact gggcagaggg gtctgctgca gaaacactgt cggctctggc tgcggagaag   3060
   ctgggcacca tggctggcct ctcaggacca ctcggatggc actcaggtgg acaggacggg   3120
   gcagggggag acttggcacc tgacctcgag ccttatttgt gaagtcctta tttcttcaca   3180
   aagaagagga acggaaatgg gacgtcttcc ttaacatctg caaacaagga ggcgctggga   3240
   tatcaaactg gaattc                                                   3256
<210> 4
   <211> 844
   <212> PRT
   <213> Homo sapiens
<400> 4
   Met Gly Pro Gly Gly Pro Cys Thr Pro Val Gly Trp Pro Leu Pro Leu
   1               5                   10                  15
   Leu Leu Val Met Ala Ala Gly Val Ala Pro Val Trp Ala Ser His Ser
               20                  25                  30
   Pro His Leu Pro Arg Pro His Pro Arg Val Pro Pro His Pro Ser Ser
           35                  40                  45
   Glu Arg Arg Ala Val Tyr Ile Gly Ala Leu Phe Pro Met Ser Gly Gly
       50                  55                  60
   Trp Pro Gly Gly Gln Ala Cys Gln Pro Ala Val Glu Met Ala Leu Glu
   65                  70                  75                  80
   Asp Val Asn Ser Arg Arg Asp Ile Leu Pro Asp Tyr Glu Leu Lys Leu
                   85                  90                  95
   Ile His His Asp Ser Lys Cys Asp Pro Gly Gln Ala Thr Lys Tyr Leu
               100                 105                 110
   Tyr Glu Leu Leu Tyr Asn Asp Pro Ile Lys Ile Ile Leu Met Pro Gly
           115                 120                 125
   Cys Ser Ser Val Ser Thr Leu Val Ala Glu Ala Ala Arg Met Trp Asn
       130                 135                 140
   Leu Ile Val Leu Ser Tyr Gly Ser Ser Ser Pro Ala Leu Ser Asn Arg
   145                 150                 155                 160
   Gln Arg Phe Pro Thr Phe Phe Arg Thr His Pro Ser Ala Thr Leu His
                   165                 170                 175
   Asn Pro Thr Arg Val Lys Leu Phe Glu Lys Trp Gly Trp Lys Lys Ile
               180                 185                 190
   Ala Thr Ile Gln Gln Thr Thr Glu Val Phe Thr Ser Thr Leu Asp Asp
           195                 200                 205
   Leu Glu Glu Arg Val Lys Glu Ala Gly Ile Glu Ile Thr Phe Arg Gln
       210                 215                 220
   Ser Phe Phe Ser Asp Pro Ala Val Pro Val Lys Asn Leu Lys Arg Gln
   225                 230                 235                 240
   Asp Ala Arg Ile Ile Val Gly Leu Phe Tyr Glu Thr Glu Ala Arg Lys
                   245                 250                 255
   Val Phe Cys Glu Val Tyr Lys Glu Arg Leu Phe Gly Lys Lys Tyr Val
               260                 265                 270
   Trp Phe Leu Ile Gly Trp Tyr Ala Asp Asn Trp Phe Lys Ile Tyr Asp
           275                 280                 285
   Pro Ser Ile Asn Cys Thr Val Asp Glu Met Thr Glu Ala Val Glu Gly
       290                 295                 300
   His Ile Thr Thr Glu Ile Val Met Leu Asn Pro Ala Asn Thr Arg Ser
   305                 310                 315                 320
   Ile Ser Asn Met Thr Ser Gln Glu Phe Val Glu Lys Leu Thr Lys Arg
                   325                 330                 335
   Leu Lys Arg His Pro Glu Glu Thr Gly Gly Phe Gln Glu Ala Pro Leu
               340                 345                 350
   Ala Tyr Asp Ala Ile Trp Ala Leu Ala Leu Ala Leu Asn Lys Thr Ser
           355                 360                 365
   Gly Gly Gly Gly Arg Ser Gly Val Arg Leu Glu Asp Phe Asn Tyr Asn
       370                 375                 380
   Asn Gln Thr Ile Thr Asp Gln Ile Tyr Arg Ala Met Asn Ser Ser Ser
   385                 390                 395                 400
   Phe Glu Gly Val Ser Gly His Val Val Phe Asp Ala Ser Gly Ser Arg
                   405                 410                 415
   Met Ala Trp Thr Leu Ile Glu Gln Pro Gln Gly Gly Ser Tyr Lys Lys
               420                 425                 430
   Ile Gly Tyr Tyr Asp Ser Thr Lys Asp Asp Leu Ser Trp Ser Lys Thr
           435                 440                 445
   Asp Lys Trp Ile Gly Gly Ser Pro Pro Ala Asp Gln Thr Leu Val Ile
       450                 455                 460
   Lys Thr Phe Arg Phe Leu Ser Gln Lys Leu Phe Ile Ser Val Ser Val
   465                 470                 475                 480
   Leu Ser Ser Leu Gly Ile Val Leu Ala Val Val Cys Leu Ser Phe Asn
                   485                 490                 495
   Ile Tyr Asn Ser His Val Arg Tyr Ile Gln Asn Ser Gln Pro Asn Leu
               500                 505                 510
   Asn Asn Leu Thr Ala Val Gly Cys Ser Leu Ala Leu Ala Ala Val Phe
           515                 520                 525
   Pro Leu Gly Leu Asp Gly Tyr His Ile Gly Arg Asn Gln Phe Pro Phe
       530                 535                 540
   Val Cys Gln Ala Arg Leu Trp Leu Leu Gly Leu Gly Phe Ser Leu Gly
   545                 550                 555                 560
   Tyr Gly Ser Met Phe Thr Lys Ile Trp Trp Val His Thr Gly Phe Thr
                   565                 570                 575
   Lys Lys Glu Glu Lys Lys Glu Trp Arg Lys Thr Leu Glu Pro Trp Lys
               580                 585                 590
   Leu Tyr Ala Thr Val Gly Leu Leu Val Gly Met Asp Val Leu Thr Leu
           595                 600                 605
   Ala Ile Trp Gln Ile Val Asp Pro Leu His Arg Thr Ile Glu Thr Phe
       610                 615                 620
   Ala Lys Glu Glu Pro Lys Glu Asp Ile Asp Val Ser Ile Leu Pro Gln
   625                 630                 635                 640
   Leu Glu His Cys Ser Ser Arg Lys Met Asn Thr Trp Leu Gly Ile Phe
                   645                 650                 655
   Tyr Gly Tyr Lys Gly Leu Leu Leu Leu Leu Gly Ile Phe Leu Ala Tyr
               660                 665                 670
   Glu Thr Lys Ser Val Ser Thr Glu Lys Ile Asn Asp His Arg Ala Val
           675                 680                 685
   Gly Met Ala Ile Tyr Asn Val Ala Val Leu Cys Leu Ile Thr Ala Pro
       690                 695                 700
   Val Thr Met Ile Leu Ser Ser Gln Gln Asp Ala Ala Phe Ala Phe Ala
   705                 710                 715                 720
   Ser Leu Ala Ile Val Phe Ser Ser Tyr Ile Thr Leu Val Val Leu Phe
                   725                 730                 735
   Val Pro Lys Met Arg Arg Leu Ile Thr Arg Gly Glu Trp Gln Ser Glu
               740                 745                 750
   Ala Gln Asp Thr Met Lys Thr Gly Ser Ser Thr Asn Asn Asn Glu Glu
           755                 760                 765
   Glu Lys Ser Arg Leu Leu Glu Lys Glu Asn Arg Glu Leu Glu Lys Ile
       770                 775                 780
   Ile Ala Glu Lys Glu Glu Arg Val Ser Glu Leu Arg His Gln Leu Gln
   785                 790                 795                 800
   Ser Arg Gln Gln Leu Arg Ser Arg Arg His Pro Pro Thr Pro Pro Glu
                   805                 810                 815
   Pro Ser Gly Gly Leu Pro Arg Gly Pro Pro Glu Pro Pro Asp Arg Leu
               820                 825                 830
   Ser Cys Asp Gly Ser Arg Val His Leu Leu Tyr Lys
           835                 840

## Description

The present invention relates to isolated polynucleotide molecules which encode a novel transmembrane G-protein coupled receptor designated GABA-B₂. The novel receptor appears to be activated by the neurotransmitter γ-amino butyric acid (GABA).

γ-amino butyric acid (GABA) is the principal inhibitory neurotransmitter in the brain, whose action is mediated by two types of receptors, GABA-A and GABA-B. GABAergic inhibitory neurons typically form short pathways (e.g. from striatum to substantia nigra and from cerebellar cortex to deep cerebellar nuclei), although at least one long pathway projecting from the posterior hypothalamus to the cerebral cortex has also been recognised. This long pathway is believed to provide a direct pathway by which limbic, emotional and visceral information may be transferred to the cortex (Vincent et al., Science 220: 1309-1311, 1993).

GABA-B receptors, such as the GABA-B1a and GABA-B1b receptors, are predominantly present in the brain where they are believed to play a major role in learning and memory. In view of these functions and the known benefit of GABA-B agonists (e.g. baclofen) in the treatment of spasticity, anxiety and depression, there is considerable interest in isolating genes encoding GABA-B receptor subtypes so as to enable the recombinant production of GABA-B receptors for the development of novel therapeutics.

International Patent Publication WO 97/46675 discloses two rat GABA-B receptors, designated GABA-B R1a and GABA-B R1b, and provide amino acid sequences of the receptors and nucleotide sequences of the receptor-encoding clones. Kaupmann *et al*. speculates on the existence of additional members of the GABA-B receptor family, but does not describe or suggest any specific receptor of the GABA-B R2 subtype or provide any amino acid or nucleotide sequence information of such.

International Patent Publication WO 98/45437 discloses isolated polynucleotides, namely Secreted Expressed Sequence Tags (sEST). However, Jacobs *et al.* does not mention or suggest any EST that is specific for a GABA-B-encoding gene, and there is no description or suggestion of any full-length gene encoding a GABA-B protein.

Bettler et al, Current Opinion in Neurobiology, 8 (3): pp. 345-350, 1998 reviews the pharmacological profiles of pre-synaptic and post-synaptic GABA-B receptor subtypes. The authors speculate on the existence of GABA-B R2 receptor subtypes that might couple to downstream effectors that are different to those coupled to GABA-B R1 receptor subtypes. However, Bettler *et al.* does not disclose any specific GABA-B R2 receptors and suggests that the cloning of GABA-B receptor subtypes is difficult.

### Summary

According to a 1^{st} aspect of the present invention, we provide an isolated polynucleotide molecule encoding a GABA-B receptor having an amino acid sequence shown as SEQ ID NO: 2 or having conservative amino acid substitutions thereto.

Preferably, the polynucleotide molecule encodes a GABA-B receptor of human origin of 941 amino acids in length.

Preferably, the polynucleotide molecule comprises a nucleotide sequence shown at nucleotides 1 to 3256 or nucleotides 140 to 2962 of SEQ ID NO: 3.

There is provided, according to a 2^{nd} aspect of the present invention, a plasmid or expression vector including a polynucleotide molecule according to the 1^{st} aspect of the invention.

We provide, according to a 3^{rd} aspect of the present invention, a host cell transformed with a polynucleotide molecule according to the 1^{st} aspect of the invention comprising a nucleotide sequence shown at nucleotides 1 to 3256 or nucleotides 140 to 2962 of SEQ ID NO: 3, or a plasmid or expression vector according to the 2^{nd} aspect of the invention.

Preferably, the cell is a mammalian or insect cell.

Preferably, the cell is a Chinese hamster ovary (CHO) cell, human embryonic kidney (HEK) 293 cell or an insect Sf9 cell.

Preferably, the cell expresses the GABA-B receptor onto the cell's surface.

We provide, according to a 4th aspect of the present invention, a human GABA-B receptor in substantially pure form wherein said receptor has an amino acid sequence shown as SEQ ID NO: 2 or having conservative amino acid substitutions thereto

The present invention, in a 5th aspect, provides an antibody or fragment thereof which specifically binds to such a human GABA-B receptor.

In a 6th aspect of the present invention, there is provided a non-human animal transformed with a polynucleotide molecule according to the 1st aspect of the invention or a plasmid or expression vector according to the 2nd aspect of the invention.

There is provided, in accordance with a 7th aspect of the present invention, a method of producing GABA-B receptors comprising culturing a host cell according to the 3rd aspect of the invention under conditions enabling the expression of GABA-B receptors and optionally recovering the receptors.

We describe an isolated polynucleotide molecule encoding a GABA-B2 receptor or a functionally equivalent fragment thereof. The encoded GABA-B2 receptor maybe characterised by the N-terminal amino acid sequence: MASPRSSGQP (SEQ ID NO: 1) and may encode a human GABA-B2 receptor of about 941 amino acids.

The isolated polynucleotide molecule encodes a GABA-B2 receptor having an amino acid sequence shown as SEQ ID NO: 2, or having conservative amino acid substitutions thereto.

The polynucleotide molecule may comprise a nucleotide sequence substantially corresponding to, or showing at least 90% (more preferably, at least 95%) homology to that shown at nucleotides 1 to 3256 or nucleotides 140 to 2962 of SEQ ID NO: 3 or any portion thereof encoding a functionally equivalent GABA-B2 receptor fragment.

The isolated polynucleotide molecule may be incorporated into plasmids or expression vectors (including viral vectors), which may then be introduced into suitable bacterial, yeast, insect and mammalian, host cells. Such host cells may be used to express the GABA-B2 receptor encoded by the isolated polynucleotide molecule.

We therefore describe a mammalian, insect, yeast or bacterial host cell transformed with the polynucleotide molecule of the first aspect.

The polynucleotide may also be used for producing GABA-B2 receptors or functionally equivalent fragments thereof in a method, comprising culturing the host cell under conditions enabling the expression of GABA-B2 receptors or functionally equivalent fragments thereof.

Preferably, the host cell is mammalian or of insect origin. Where the cell is mammalian, it is presently preferred that it be a Chinese hamster ovary (CHO) cell, monkey kidney (COS) cell or human embryonic kidney 293 cell. Where the cell is of insect origin, it is presently preferred that it be an insect Sf9 cell.

In a preferred embodiment, the GABA-B2 receptors or fragments thereof are expressed onto the surface of the host cell.

By using the polynucleotide molecule it is possible to obtain GABA-B2 receptor protein or fragments thereof in a substantially pure form.

Accordingly, we describe a GABA-B2 receptor or a functionally equivalent fragment of said receptor, in a substantially pure form.

We also describe an antibody capable of specifically binding to the GABA-B2 receptor. Such antibodies may be produced by any of the methods routine to the art.

A non-human animal may also be transformed with such a polynucleotide molecule.

We describe a method for detecting agonist or antagonist agents of a GABA-B2 receptor, comprising contacting a GABA-B2 receptor, functionally equivalent fragment thereof or a cell transfected with and expressing the DNA molecule, with a test agent under conditions enabling the activation of a GABA-B2 receptor, and detecting an increase or decrease in activity of the GABA-B2 receptor or functionally equivalent fragment thereof.

An increase or decrease in activity of the receptor or functionally equivalent fragment thereof may be detected by measuring changes in cAMP production, Ca²⁺ levels or IP3 turnover after activating the receptor molecule with specific agonists or antagonists.

We describe an oligonucleotide or polynucleotide probe comprising a nucleotide sequence of 10 or more nucleotides, the probe comprising a nucleotide sequence such that the probe specifically hybridises to the polynucleotide molecule of the first aspect under high stringency conditions (Sambrook et al., Molecular cloning: a laboratory manual, Second Edition, Cold Spring Harbor Laboratory Press).

An antisense oligonucleotide or polynucleotide molecules comprising a nucleotide sequence capable of specifically hybridising to an mRNA molecule which encodes a GABA-B2 receptor so as to prevent translation of the mRNA molecule are also described.

Such antisense oligonucleotide or polynucleotide molecules may include a ribozyme region to catalytically inactivate mRNA to which it is hybridised.

The polynucleotide molecule may be a dominant negative mutant which encodes a gene product causing an altered phenotype by, for example, reducing or eliminating the activity of endogenous GABA-B2 receptors.

The term "substantially corresponding" as used herein in relation to amino acid sequences is intended to encompass minor variations in the amino acid sequences which do not result in a decrease in biological activity of the GABA-B2 receptor. These variations may include conservative amino acid substitutions. The substitutions envisaged are:-
G, A, V, I, L, M; D, E; N, Q; S, T; K, R, H; F, Y, W, H; and P, Nα-alkalamino acids.

The term "substantially corresponding" as used herein in relation to nucleotide sequences is intended to encompass minor variations in the nucleotide sequences which due to degeneracy in the DNA code do not result in a change in the encoded protein. Further, this term is intended to encompass other minor variations in the sequence which may be required to enhance expression in a particular system but in which the variations do not result in a decrease in biological activity of the encoded protein.

The term "functionally equivalent fragment/s" as used herein is intended to refer to fragments of the GABA-B2 receptor that exhibit binding specificity and activity that is substantially equivalent to the GABA-B2 receptor from which it/they is/are derived.

Throughout this specification, unless the context requires otherwise, the word "comprise", or variations such as "comprises" or "comprising", will be understood to imply the inclusion of a stated element, integer or step, or group of elements, integers or steps, but not the exclusion of any other element, integer or step, or group of elements, integers or steps.

Reference to percent homology made in this specification have been calculated using the BLAST program blastn as described by Altschul, SF. et al., "Capped BLAST and PSI-BLAST: a new generation of protein database search programs", Nucleic Acids Research, Vol. 25, No. 17, pp. 3389-3402 (1997).

### Brief description of the accompanying Figures:

Figure 1 provides the nucleotide sequence of a cDNA encoding the human GABA-B2 receptor and includes the predicted amino acid sequence.

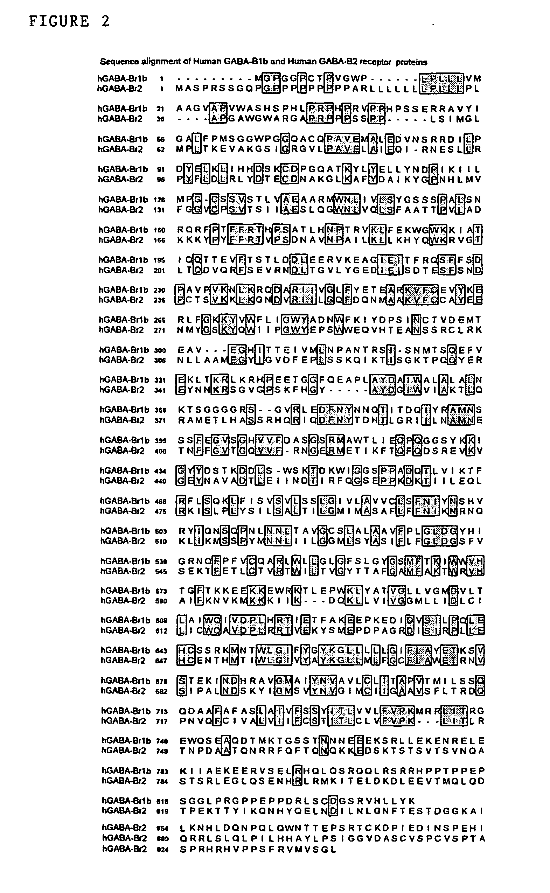

Figure 2 shows the degree of identity between the predicted amino acid sequence of the human GABA-B2 and GABA-1b receptors.

### Detailed Disclosure of the Invention:

### Human GABA-B2 receptor cDNA

A human hippocampus cDNA library (Stratagene) was screened under low stringency conditions with a 184 bp ³²P-labelled fragment (corresponding to nucleotides 2051 to 2235 of SEQ ID NO: 3) originated from a human brain EST clone (z43654). A cDNA clone encoding a complete human GABA-B gene was obtained from the screen. The nucleotide sequence of the cDNA clone is shown as SEQ ID NO: 3 and encodes a protein of 941 amino acids (SEQ ID NO: 2) consisting of a large extracellular domain (amino acids 1 to 480) followed by 7 hydrophobic regions (amino acids 481 to 494, 518 to 545, 558 to 578, 597 to 618, 653 to 676, 691 to 713 and 719 to 743) typical of G-protein coupled receptors.

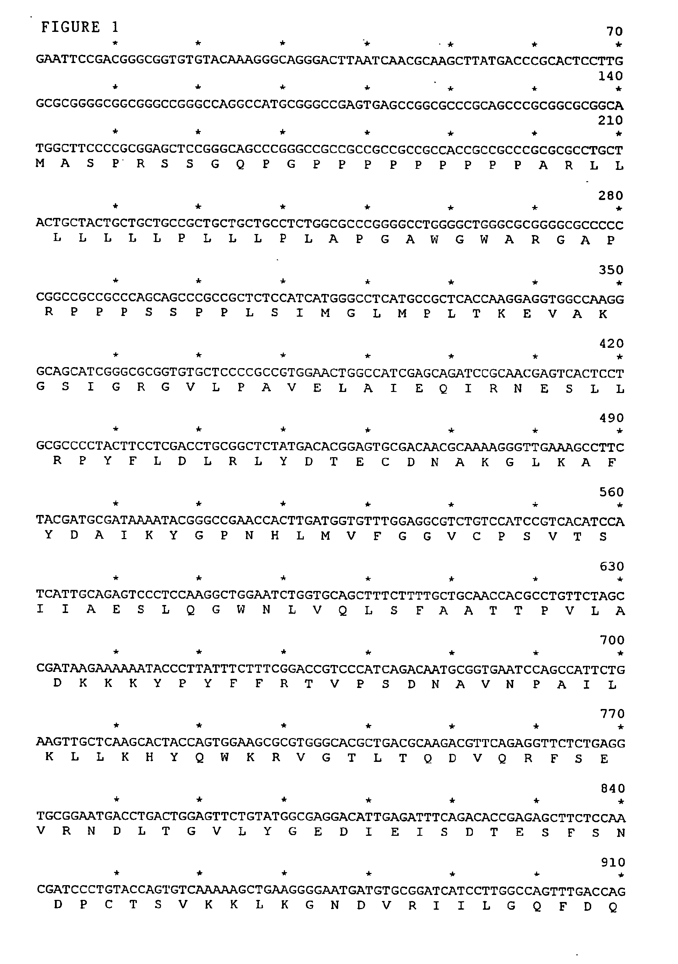

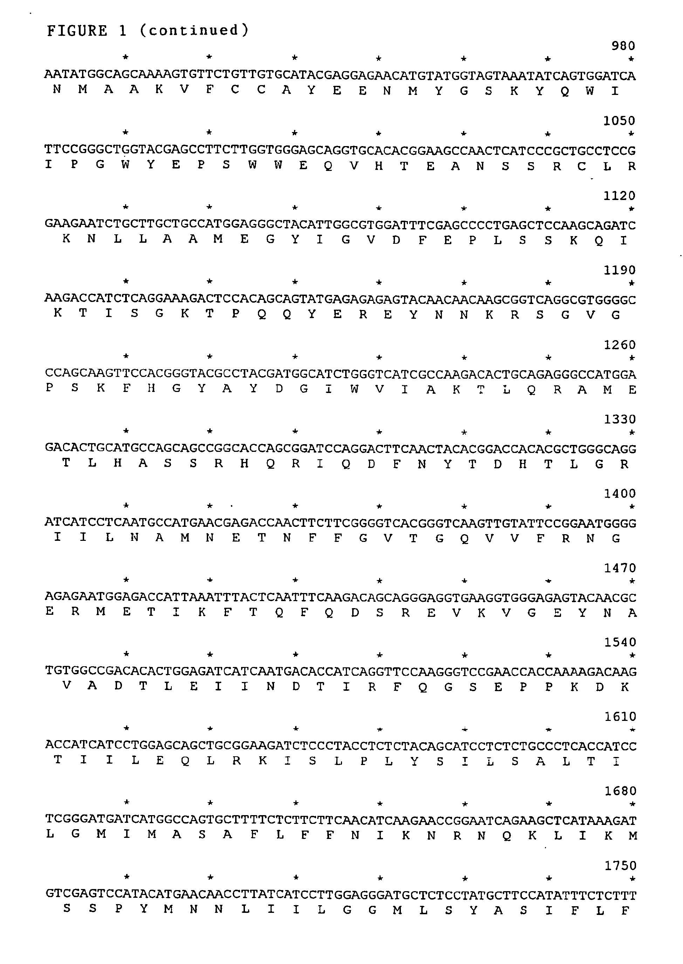

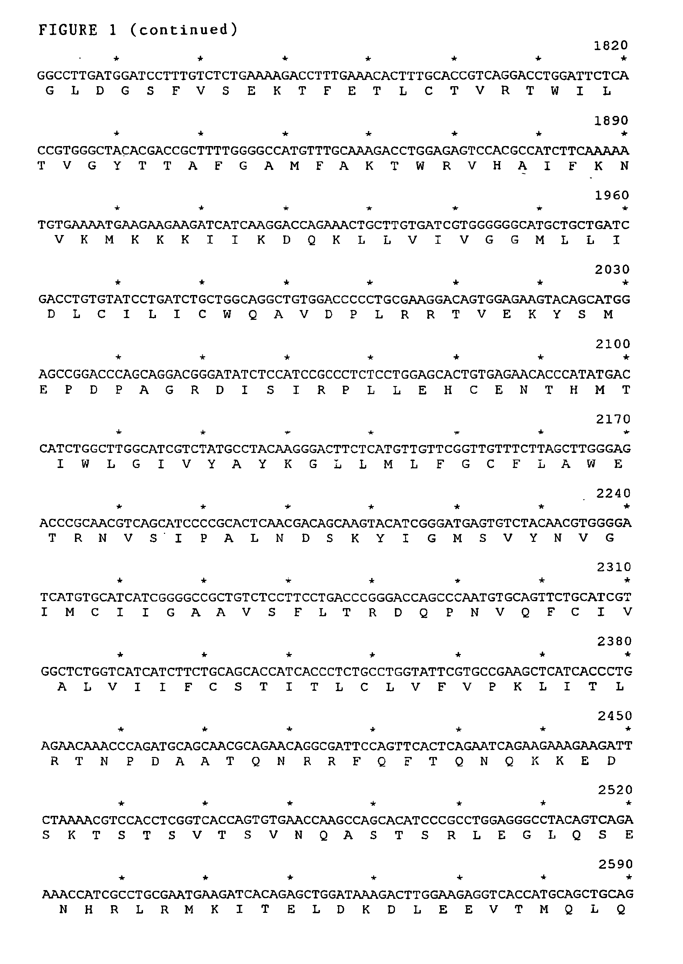

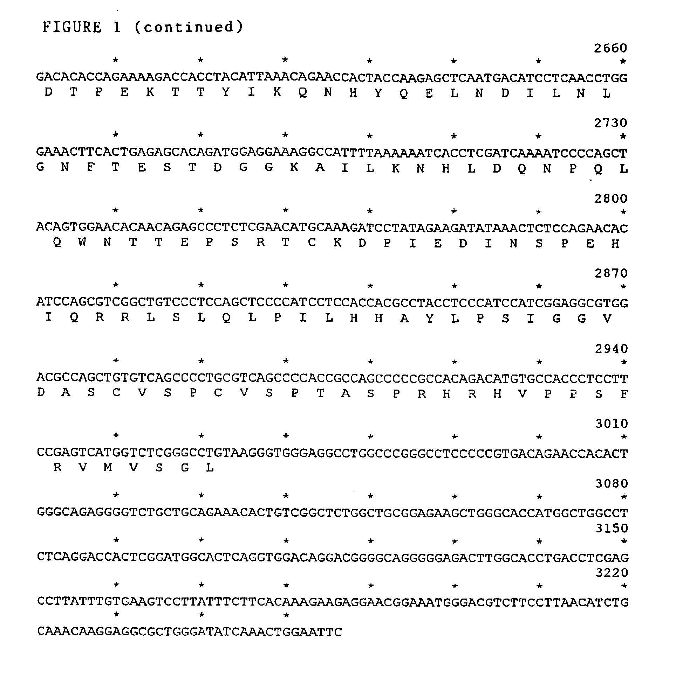

Sequence comparison with other G-protein coupled receptors identify GABA-B1a, GABA-B1b (Figure 1) and metabotropic glutamate receptors (Kaupman, K. et al., Nature 386: 239-246, 20 March 1997 and Pin, J. et al., Neuropharmacology 34: 1-26, 1995) as the most closely related group.

Northern analysis has identified brain as the tissue with the highest expression of the human GABA-B2 mRNA. In particular, Northern analysis experiments using multiple tissue Northern blots (Clontech) identified high levels of expression of the human GABA-B2 mRNA in the cerebellum, cerebral cortex, occipital pole, frontal lobe and temporal lobe. Lower levels of expression were seen in the thalamus, amygldala, hippocampus, substantia nigra, putamen, subthalamic nucleus, caudate nucleus, and medulla. No apparent expression was seen in the spinal cord and corpus callosum.

Further, *in situ* hybridisation of rat brain sections has identified discrete areas of expression in the hippocampus, amygdala, the piriform cortex and also the hypothalamus. This mRNA distribution is consistent with the expression of other subtypes of the GABA-B receptor family.

### Chromosomal Localisation of Human GABA-B2 receptor gene

In order to determine the chromosomal localisation of the human GABA-B2 receptor gene, the complete cDNA clone was nick-translated with biotin-14-dAPT and hybridised *in situ* at a final concentration of 5 ng/ml to metaphase chromosomal spreads from two normal males. The fluoerescence *in situ* hybridisation (FISH) method was modified from that previously described (Callen, DF *et al., Ann Genet* 33: 219-221 1990) in that chromosomes were stained before analysis with both propidium iodide (as counterstain) and DAPI (for chromosome identification). Images of metaphase preparations were captured by a CCD camera and computer enhancement software.

Twenty metaphases from a first normal male were examined for fluorescent signal. All of these metaphases showed signal on one or both chromatids of chromosome 9 in the region 9q21. There was a total of 7 nonspecific background dots observed in these 20 metaphases. A similar result was obtained from hybridisation of the probe to 20 metaphases from a second normal male.

### Sequence Listings:

Applicant: Garvan Institute of Medical Research
   Title of Invention: Novel GABA-B Receptor
Prior Application Number: PP4384
   Prior Application Filing Date: 1998-06-29
Number of SEQ ID NOs: 4
Software: PatentIn Ver. 2.1
SEQ ID NO: 1
   Length: 10
   Type: PRT
   Organism: Homo sapiens
Sequence: 1
SEQ ID NO: 2
   Length: 941
   Type: PRT
   Organism: Homo sapiens
Sequence: 2
SEQ ID NO: 3
   Length: 3256
   Type: DNA
   Organism: Homo sapiens
Sequence: 3
SEQ ID NO: 4
   Length: 844
   Type: PRT
   Organism: Homo sapiens
Sequence: 4

## Claims

1. An isolated polynucleotide molecule encoding a GABA-B receptor having an amino acid sequence shown as SEQ ID NO: 2 or having conservative amino acid substitutions thereto.

2. A polynucleotide molecule according to Claim 1, wherein the polynucleotide molecule encodes a GABA-B receptor of human origin of 941 amino acids in length.

3. A polynucleotide molecule according to Claim 1 or 2, wherein the polynucleotide molecule comprises a nucleotide sequence shown at nucleotides 1 to 3256 or nucleotides 140 to 2962 of SEQ ID NO: 3.

4. A plasmid or expression vector including a polynucleotide molecule according to any one of Claims 1 to 3.

5. A host cell transformed with a polynucleotide molecule according to Claim 3 or a plasmid or expression vector according to Claim 4.

6. A host cell according to Claim 5, wherein the cell is a mammalian or insect cell.

7. A host cell according to Claim 6, wherein the cell is a Chinese hamster ovary (CHO) cell, human embryonic kidney (HEK) 293 cell or an insect Sf9 cell.

8. A host cell according to any one of Claims 5 to 7, wherein the cell expresses the GABA-B receptor onto the cell's surface.

9. A human GABA-B receptor in a substantially pure form wherein said receptor has an amino acid sequence shown as SEQ ID NO: 2 or having conservative amino acid substitutions thereto.

10. An antibody or fragment thereof which specifically binds to a human GABA B receptor according to claim 9.

11. A non-human animal transformed with a polynucleotide molecule according to any one of claims 1 to 3 or a plasmid or expression vector according to claim 4.

12. A method of producing GABA-B receptors comprising culturing a host cell according to any one of claims 5 to 8 under conditions enabling the expression of GABA-B receptors and optionally recovering the receptors.

## Patentansprüche

1. Isoliertes Polynukleotidmolekül, welches einen GABA-B-Rezeptor codiert, der eine Aminosäuresequenz aufweist, die als SEQ ID NO: 2 gezeigt ist oder die darin konservative Aminosäuresubstitutionen hat.

2. Polynukleotidmolekül nach Anspruch 1, wobei das Polynukleotidmolekül einen GABA-B-Rezeptor menschlichen Ursprungs mit einer Länge von 941 Aminosäuren codiert.

3. Polynukleotidmolekül nach Anspruch 1 oder 2, wobei das Polynukleotidmolekül eine Nukleotidsequenz, die an den Nukleotiden 1 bis 3256 oder den Nukleotiden 140 bis 2962 von SEQ ID NO: 3 gezeigt ist.

4. Plasmid- oder Expressionsvektor, welcher ein Polynukleotidmolekül nach einem der Ansprüche 1 bis 3 beinhaltet.

5. Wirtszelle, transformiert mit einem Polynukleotidmolekül nach Anspruch 3 oder einem Plasmid- oder Expressionsvektor nach Anspruch 4.

6. Wirtszelle nach Anspruch 5, wobei die Zelle eine Säuger- oder eine Insektenzelle ist.

7. Wirtszelle nach Anspruch 6, wobei die Zelle eine Eizelle des chinesischen Hamsters (CHO-Zelle), eine menschliche embryonale Nieren- (HEK-) 293-Zelle oder eine Sf9-Insektenzelle ist.

8. Wirtszelle nach einem der Ansprüche 5 bis 7, wobei die Zelle den GABA-B-Rezeptor auf der Zelloberfläche exprimiert.

9. Humaner GABA-B-Rezeptor in im wesentlichen reiner Form, wobei der Rezeptor eine Aminosäuresequenz aufweist, die als SEQ ID NO: 2 gezeigt ist oder die darin konservative Aminosäuresubstitutionen hat.

10. Antikörper oder Fragment davon, der bzw. das spezifisch an einen humanen GABA-B-Rezeptor nach Anspruch 9 bindet.

11. Nicht-menschliches Tier, transformiert mit einem Polynukleotidmolekül nach einem der Ansprüche 1 bis 3 oder einem Plasmid- oder Expressionsvektor nach Anspruch 4.

12. Verfahren zum Herstellen von GABA-B-Rezeptoren, welches das Kultivieren einer Wirtszelle nach einem der Ansprüche 5 bis 8 unter Bedingungen, die die Expression von GABA-B-Rezeptoren erlauben, und optional das Gewinnen der Rezeptoren umfaßt.

## Revendications

1. Molécule de polynucléotide isolée codant pour un récepteur GABA-B ayant une séquence d'aminoacides représentée par la SEQ ID N° 2 ou comportant des substitutions d'aminoacides conservatrices dans cette séquence.

2. Molécule de polynucléotide suivant la revendication 1, ladite molécule de polynucléotide codant pour un récepteur GABA-B d'origine humaine, de 941 aminoacides de longueur.

3. Molécule de polynucléotide suivant la revendication 1 ou 2, ladite molécule de polynucléotide comprenant une séquence de nucléotides représentée par les nucléotides 1 à 3256 ou les nucléotides 140 à 2962 de la SEQ ID N° 3.

4. Plasmide ou vecteur d'expression comprenant une molécule de polynucléotide suivant l'une quelconque des revendications 1 à 3.

5. Cellule hôte transformée avec une molécule de polynucléotide suivant la revendication 3 ou un plasmide ou vecteur d'expression suivant la revendication 4.

6. Cellule hôte suivant la revendication 5, ladite cellule étant une cellule de mammifère ou une cellule d'insecte.

7. Cellule hôte suivant la revendication 6, ladite cellule étant une cellule d'ovaire de hamster chinois (CHO), une cellule de rein embryonnaire humain (HEK) 293 ou une cellule d'insecte SF9.

8. Cellule hôte suivant l'une quelconque des revendications 5 à 7, ladite cellule exprimant le récepteur GABA-B sur la surface de la cellule.

9. Récepteur GABA-B humain sous une forme substantiellement pure, ledit récepteur ayant une séquence d'aminoacides représentée par la SEQ ID N° 2 ou comportant des substitutions d'aminoacides conservatrices dans cette séquence.

10. Anticorps ou son fragment qui se lie spécifiquement à un récepteur GABA-B humain suivant la revendication 9.

11. Animal non humain transformé avec une molécule de polynucléotide suivant l'une quelconque des revendications 1 à 3 ou un plasmide ou vecteur d'expression suivant la revendication 4.

12. Procédé pour la production de récepteurs GABA-B, comprenant la culture d'une cellule hôte suivant l'une quelconque des revendications 5 à 8 dans des conditions permettant l'expression des récepteurs GABA-B et, facultativement, la récupération des récepteurs.
